# EUROPÄISCHE PATENTSCHRIFT

(11) **EP 0 604 774 B1**
(45) Veröffentlichungstag und Bekanntmachung des Hinweises auf die Patenterteilung: **29.01.1997**
(21) Anmeldenummer: 93119286.8
(22) Anmeldetag: 30.11.1993
(51) Int. Cl.: A61K 33/08, A61K 31/23, A61K 6/00

(54) **Verwendung eines Gemischs aus Calciumhydroxid und Oleum pedum tauri zur Kollagenneubildung in vivo**
Use of mixture of calciumhydroxide and oleum pedum tauri at collagensynthesis in vivo
Utilisation d'un mélange de l'hydroxide de calcium et d'oleum pedum tauri dans la production de collagène in vivo

(30) Priorität: 03.12.1992 DE 4240713
(43) Veröffentlichungstag der Anmeldung: 06.07.1994
(73) Patentinhaber: Dietz, Georg, Prof. Dr., D-81925 München (DE)
(72) Erfinder: Dietz, Georg, Prof. Dr., D-81925 München (DE)
(74) Vertreter: Henkel, Feiler, Hänzel & Partner

(56) Entgegenhaltungen:
- DE-C- 2 932 738
- US-A- 4 855 136
- H.v. CZETSCH-LINDENWALD/H.P. FIEDLER; "Hilfsstoffe fUr Pharmazie und angrenzende Gebiete", 2. Auflage, Band 3 EDITIO CANTOR K.G., Aulendorf i. WUrttemberg, 1963 Seiten 271-272 * Abschnitt "Rinder- klauen!l" *
- H. v. CZETSCH-LINDENWALD/H.P. FIEDLER: "Hilfsstoffe fUr Pharmazie und angrenzende Gebiete", 2. Auflage, Band 3, EDITIO CANTOR K.G., Aulendorf/WUrttemberg, 1963, Seiten 271-272

## Beschreibung

Die Erfindung betrifft die Verwendung eines Gemischs aus Calciumhydroxid und Oleum pedum tauri sowie gegebenenfalls pharmazeutisch verträglichen Hilfsstoffen bei der Herstellung eines Medikaments zur Kollagenneubildung in vivo sowie die Verwendung eines Gemischs aus Calciumhydroxid und Oelum pedum tauri bei der Herstellung eines Medikaments zur Förderung der Kollagenneubildung in vivo.

Der Knochen besteht zu etwa 60 % aus mineralischer Substanz (Hydroxylapatit, Calciumphosphat) und etwa 40 % aus organischem Material, vor allem Kollagen. Der Knochenstoffwechsel wird hauptsächlich durch das Zusammenspiel von Knochen aufbauenden Zellen (Osteoblasten) und Knochen abbauenden Zellen (Osteoklasten und Osteozyten), deren Aktivitäten im gesunden Knochen in einem ausgewogenen Verhältnis stehen, bestimmt.

Die Knochenbildung läßt sich in zwei Hauptphasen gliedern, (a) die Synthese von organischem Gewebe (Kollagen-Synthese) und (b) die daran anschließende und durch sogenannte Matrixvesikel vermittelte Einlagerung von mineralischer Substanz in die vorgegebene organische Matrix.

Das Bindegewebsprotein Kollagen macht den größten Anteil der organischen Substanz des Knochens aus. Das Protein besteht aus drei helikal gewundenen Polypeptidketten, deren Aminosäurezusammensetzung variieren kann, was zu einer Vielfalt einzelner Kollagentypen führt. Allen Kollagentypen gemeinsam ist eine außerordentlich hohe mechanische Festigkeit der Kollagenfaser. Diese Festigkeit beruht auf einer Vielzahl von intra- und intermolekularen Bindungen der Kollagenfasern, welche auf diese Weise das dichte Kollagenfaser-Netzwerk des Bindegewebes bilden. Knochengewebe wird - wie bereits erwähnt - durch Einlagerung von mineralischen Substanzen (Hydroxylapatit und Calciumphosphat) in dieses Netzwerk gebildet. Jedem Knochenaufbau infolge von Wachstums- oder Regenerationsprozessen geht eine Kollagenbiosynthese voran.

Bislang überläßt man bei Knochen-Traumen beliebiger Genese den Knochenneubildungsprozeß sich selbst, unterstützt ihn allenfalls mit Antibiotica und Corticoiden, um einer den Heilungsprozeß störenden eventuellen Infektionsgefahr vorzubeugen.

Es sind auch mehrere Faktoren beschrieben worden, welche die Knochenbildung und -regeneration beeinflussen können. Hauptsächlich handelt es sich hierbei um physikalische Faktoren (mechanische und elektrische Kräfte), Hormone (zum Beispiel Parathormon, Calcitonin, Insulin, Glucocorticoide, 1,25,OH, D3) und eine nicht genau umrissene Gruppe von Wachstumsfaktoren mit Proteincharakter (Osteochinin, Osteonektin, "insulinartige Wachstumsfaktoren") - vgl. S. Wallach, L.V. Avioli, J.H. Carstens jun "Factors in Bone Formation", Calcified Tissue International 45: 4-6 (1989)). Der Einfluß der Wasserstoffionen-Konzentration (pH-Wert) auf die Stoffwechselprozesse bei der Knochenregeneration wurden bislang noch nicht ausreichend untersucht.

Der Erfindung lag die Aufgabe zugrunde, eine Möglichkeit zur gezielten externen Beeinflussung des Knochenneubildungs- oder -regenerationsprozesses durch Stimulierung oder Einleitung der Kollagenneubildung anzugeben.

Es hat sich nun überraschenderweise gezeigt, daß bei Verwendung eines Gemischs aus Calciumhydroxid und Oleum pedum tauri sowie gegebenenfalls pharmazeutisch verträglichen Hilfsstoffen in bzw. bei Knochen-Traumen eine rasche und in ihrem Umfang erhebliche Kollagenneubildung in vivo erfolgt.

Bariumsulfathaltige Gemische aus Calciumhydroxid und Oleum pedum tauri wurden in der Zahnmedizin als Wurzelfüllpaste verwendet (DE-PS 29 32 738). Gemische aus Carboxilatzement, Calciumhydroxid und Oleum pedum tauri wurden ebenfalls in der Zahnmedizin bereits als temporäre Befestigungsmittel für provisorische Zahnstumpfabdeckungen verwendet (DE-PS 34 13 864). Aufgabe des Calciumhydroxids ist in ersterem Fall, das saure Milieu in den Wurzelkanälen ins Alkalische umzustimmen mit der Folge der Beseitigung von Entzündungen und allmählicher Bildung einer Hartgewebsbarriere. In letzterem Falle wird die Pulpitis-prophylaktische Wirkung von Calciumhydroxid ausgenutzt. Das Oleum pedum tauri dient in beiden Fällen als Anteigmittel, um einerseits eine einfache und vollständige Füllung der Wurzelkanäle mit dem eigentlichen Wirkstoff Calciumhydroxid (und dem Konstrastmittel Bariumsulfat) zu gewährleisten und andererseits die Aushärtung des temporären Befestigungsmittels für provisorische Zahnstumpfabdeckungen soweit zu verlangsamen, daß das Calciumhydroxid noch durch die feinen Dentinkanälchen zur Pulpa vordringen und dort seine Wirkung entfalten kann. In beiden Literaturstellen findet sich nicht der geringste Hinweis darauf, daß die Mischung aus Calciumhydroxid und Oleum pedum tauri eine massive Kollagenneubildung als Voraussetzung für eine Knochenregeneration zu induzieren vermag.

In dem erfindungsgemäßen Gemisch aus Calciumhydroxid und Oleum pedum tauri kann das Verhältnis Calciumhydroxid zu Oleum pedum tauri 2/1 bis 1/2, vorzugsweise 1/1, betragen. Eine Abweichung vom bevorzugten Mischungsverhältnis kann durch die speziellen Gegebenheiten des Wund-Traumas erforderlich sein.

Wenn das erfindungsgemäße Gemisch aus Calciumhydroxid und Oleum pedum tauri eine besonders geschmeidige und glatte Konsistenz aufweisen soll, kann ihm auch noch weiße Vaseline einverleibt werden.

Obwohl üblicherweise der Knochenheilungs- oder -regenerationsprozeß nicht röntgenologisch überwacht zu werden braucht, kann dies doch in manchen Fällen angezeigt sein. Für diesen Fall wird dem erfindungsgemäßen Gemisch aus Calciumhydroxid und Oleum pedum tauri noch Bariumsulfat als Röntgenkontrastmittel einverleibt. Da jedoch das Bariumsulfat - wie noch gezeigt werden wird - die Kollagenneubildung etwas schwächer ausfallen läßt, setzt man dem erfindungsgemäßen Gemisch im Bedarfsfall gerade so viel Bariumsulfat zu, daß das Gemisch eben röntgensichtbar wird.

Die wesentlichen Bestandteile des erfindungsgemäßen Gemischs, nämlich Calciumhydroxid und Oleum pedum tauri, sollten zur Vermeidung einer Verseifung des Oleum pedum tauri erst mehr oder minder unmittelbar vor der Applikation auf einem oder in ein Knochen-Trauma gemischt werden. Dies kann beispielsweise durch getrenntes Verpacken der beiden Bestandteile in sogenannten Rüttelkapseln und Vereinigen der beiden Bestandteile unmittelbar vor Gebrauch erfolgen.

Die Applikation des erfindungsgemäßen Gemischs auf oder in das Knochen-Trauma kann - je nach seiner Konsistenz - mittels Spritzen, Spateln oder Pinseln erfolgen.

Für das erfindungsgemäße Gemisch gibt es zahlreiche Einsatzmöglichkeiten in der allgemeinen Chirurgie und Kieferchirurgie, Orthopädie, Implantologie, Traumatologie und dergleichen, da das erfindungsgemäße Gemisch auf oder in gewebige Knochen-Traumen, wie Frakturflächen, Bohrungen, Cavitäten und dergleichen applizierbar ist und am jeweiligen Applikationsort sofort eine in vivo-Kollagenneubildung induziert.

Da bekanntlich in manchen einschlägigen medizinischen Disziplinen mit metallischen Fixiermitteln gearbeitet wird, empfiehlt es sich in diesem Falle, die für das Einsetzen des Fixiermittels vorbereitete Bohrung vor dem Einsetzen des Fixiermittels mit dem erfindungsgemäßen Gemisch zu füllen und dann erst das Fixiermittel einzuführen. Dadurch kann man dem bei derartigen Maßnahmen unvermeidlichen primären Knochenschwund begegnen und dadurch die Ein- oder Anpassung des Fixiermittels in bzw. an das umgebende Knochengewebe und die Fixierung des Fixiermittels selbst durch das Knochengewebe beschleunigen.

Überschüssiges Gemisch stört hierbei im übrigen nicht, da es beim Einbringen des Fixiermittels in die mit dem Gemisch gefüllte Bohrung entweder wieder herausgedrückt wird oder in die Spongiosa diffundiert.

Es dürfte selbstverständlich sein, daß das erfindungsgemäße Gemisch bzw. seine Bestandteile sowohl steril verpackt als auch appliziert werden müssen.

In höchst überraschender Weise hat es sich auch noch gezeigt, daß das erfindungsgemäße Gemisch auch ohne Antibiotikum- und/oder Corticoid-Unterstützung einer durch das Knochen-Trauma bedingten Entzündungsreaktion entgegenwirkt bzw. diese rasch zum Abklingen bringt. Seine einfache Zusammensetzung und ausgeprägte Wirksamkeit bei in vivo-Kollagenneubildung unter gleichzeitiger Entzündungshemmung machen das erfindungsgemäße Gemisch zu einem in Zukunft unverzichtbaren Mittel in der Knochen-Traumatologie.

Die folgenden Beispiele sollen die Erfindung näher veranschaulichen.

### Beispiel 1

A) Zunächst soll die Wechselwirkung zwischen dem erfindungsgemäßen Gemisch aus Calciumhydroxid und Oleum pedum tauri und Gewebe aufgeklärt werden. So sind Daten über die Verteilung des erfindungsgemäßen Gemischs aus Calciumhydroxid und Oleum pedum tauri im Knochengewebe die Voraussetzung, um Hypothesen über einen möglichen Wirkmechanismus des Medikaments aufstellen zu können. Daher sind Experimente an Gewebekulturen sinnvoller als solche an Zellkulturen, da es erst in einer Gewebekultur möglich ist, Zell-Zell-Interaktionen zu studieren.

### 1. Material und Methoden

### 1.1. Gewebematerial

Menschliches Knochengewebe, welche bei Osteotomien anfiel, wurde von Kliniken zur Verfügung gestellt.

Embryonales Knochengewebe wurde aus 10 bis 17 Tage alten Hühnerembryonen (Gallus domesticus) gewonnen.

### 1.2. Gewebekultur

Unmittelbar nach Entnahme wurde das Gewebe in das Transportmedium überführt. Es wurden unter sterilen Bedingungen etwa 2 mm³ große Knochenfragmente präpariert und nach Bestimmung des Gewichts direkt für die Experimente eingesetzt.

Für die Gewebekultur wurde die Earl'sche Modifikation des Minimal Essential Medium (MEM) nach Eagle mit 20 mM Hepes-Puffer verwendet.

Dem Medium wird vor Versuchsbeginn 4 % fötales Kälberserum und 1 % Antibiotika-Lösung (Penicillin/Streptomycin/Amphotericin B) zugesetzt, für die Markierungsexperimente zusätzlich 1 mM beta-Aminopropionidyl, 2 mM Na-Ascorbat und 2 bis 10 µ Ci-Isotope (¹⁴ C-Prolin). Kultiviert wird in 25 ml Erlenmeyer-Kolben bei 37° C im Schüttelwasserbad bei kleinster Frequenz.

### 1.3 Bestimmung der Atmungsaktivität

Die Atmungsaktivität ist ein sensibler Marker für die Stoffwechselaktivität des Gewebes. Schon geringste Änderungen des physiologischen Zustandes des Gewebes schlagen sich in einer meßbaren Änderung der Atmungsaktivität nieder.

Für die Bestimmung der Atmungsaktivität wurde ein Clark-Sensor (Platin/Silber-Elektroden in gesättigter Kaliumchlorid-Lösung) benutzt. Beim Anlegen einer Spannung von 0,8 V an die Elektrode ist der Sauerstoffreduktionsstrom direkt proportional dem Sauerstoffpartialdruck in der Meßlösung (Kulturmedium). Die Zufuhr von O₂-gesättigtem Medium bei Unterschreitung eines bestimmten Sauerstoffpartialdrucks und die Datenauswertung erfolgen rechnergesteuert.

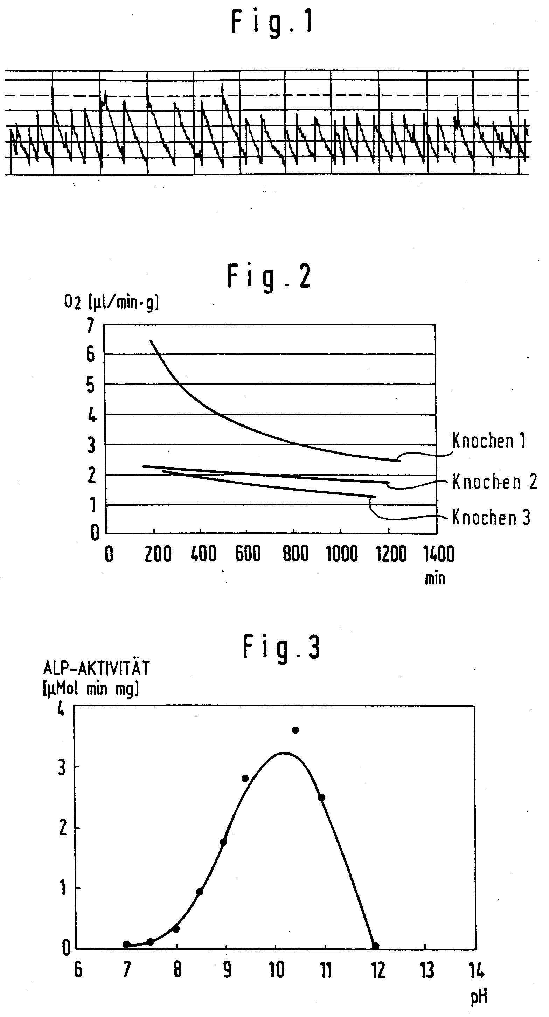

Knochengewebe besitzt typischerweise eine Atmungsaktivität von 2 - 3 µl O₂ x min⁻¹ x g⁻¹. Die Atmungsaktivität liegt damit größenordnungsmäßig im Bereich der Atmungsaktivität von ruhendem Muskelgewebe. Eine typische Atmungskurve für Knochengewebe zeigt Fig. 1. Der sägezahnartige Verlauf der Atmungskurve gemäß Fig. 1 ergibt sich aus der Tatsache, daß bei Unterschreitung eines bestimmten O₂-Partialdrucks in der Meßlösung frisches, O₂-gesättigtes Medium zugeführt wird.

Fig. 2 zeigt durchschnittliche O₂-Verbrauchswerte aus drei Messungen. Der Sauerstoffverbrauch von embryonalem Knochengewebe (Gallus domesticus) wurde in einer Gewebekultur mit Hilfe des Clark-Sensors bestimmt. Der Sauerstoffverbrauch liegt zwischen 3 und 5 µl O₂ x min⁻¹ x g⁻¹. Die Atmungsaktivität nimmt im Laufe der Zeit um etwa 50 % ab, was bei Gewebekulturen durchaus normal ist.

### 1.4 Enzymtests

Ein Enzym, welches eng mit der Mineralisation des Knochengewebes in Zusammenhang gebracht wird, ist alkalische Phosphatase. Dieses Enzym ist schon seit längerem charakterisiert, über die Funktion des Enzyms bei der Mineralisierung wird noch diskutiert. Da ein enger Zusammenhang zwischen der Osteoblasten-Tätigkeit und der Aktivität von alkalischer Phosphatase besteht, kann alkalische Phosphatase als Marker der Osteblastenaktivität angesehen werden. Bei Skelettwachstum im Jugendalter, bei der Knochenregeneration und bei Erkrankungen des Knochenstoffwechsels werden erhöhte Aktivitätswerte von alkalischer Phosphatase im Blutserum gefunden.

Die Aktivität von alkalischer Phosphatase wurde in einem Rohextrakt bestimmt. Dafür wurden 500 mg Gewebe mit 1 ml Aufschlußpuffer versetzt und mit einem Messer zerkleinert. Anschließend wurden 500 mg Mahlperlen zugegeben und 20 min aufgeschlossen. Nach Zentrifugation wurde der Rohextrakt für die Messungen eingesetzt.

Alkalische Phosphatase wird anhand des Umsatzes von p-Nitrophenylphosphat zu Nitrophenol und Phosphat nachgewiesen. Das bei der Hydrolyse entstehende Nitrophenol ist gelb und kann daher im Photometer bei einer Wellenlänge von 410 nm nachgewiesen werden.

Die Fig. 3 zeigt die pH-Abhängigkeit der Aktivität von alkalischer Phosphatase. Die Aktivität von alkalischer Phosphatase aus Knochen wurde anhand des Umsatzes von p-Nitrophenylphosphat bestimmt. Das Aktivitätsmaximum liegt bei pH 10,5. Bei physiologischem pH von 7 besitzt alkalische Phosphatase nur etwa 1 % der maximalen Aktivität.

### 1.5 pH-Bestimmung

Ein erfindungsgemäßes Gemisch aus Calciumhydroxid und Oleum pedum tauri bzw. eine wäßrige Calciumhydroxid-Suspension wurden mit 30 ml Imidazol/HCl-Puffer (1 mM pH 7) überschichtet, worauf der pH-Wert in der Lösung mit Hilfe einer pH-Elektrode kontinuierlich verfolgt wurde.

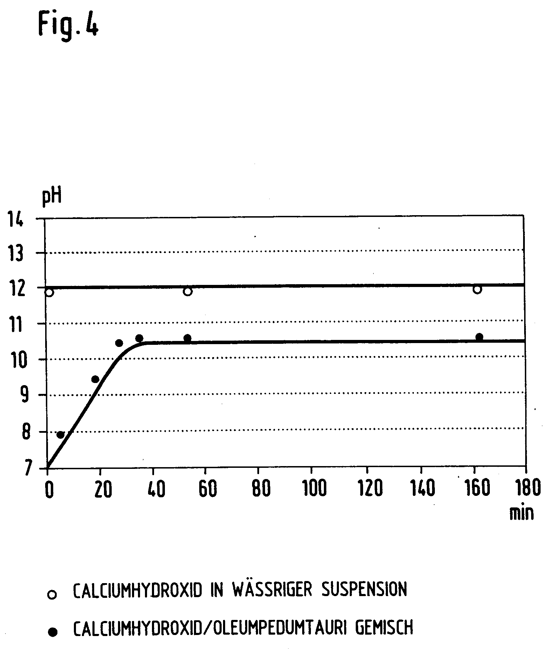

Bei diesen Messungen zeigte es sich, daß Calciumhydroxid in Oelum pedum tauri grundlegend andere Eigenschaften besitzt als Calciumhydroxid in wäßriger Suspension. Die Fig. 4 zeigt, daß eine wäßrige Calciumhydroxid-Suspension einen sofortigen pH-Sprung auf pH 12 verursacht, und daß bei dem erfindungsgemäßen Calciumhydroxid/Oleum pedum tauri-Gemisch ein langsamer Anstieg bis zu einem pH-Wert von größer 10 erfolgt.

### 1.6 Kollagenbestimmung

Der Hauptteil der organischen Substanz im Knochen besteht - wie bereits erwähnt - aus Kollagen, einem Bindegewebsprotein. Wachstums- und Regenerationsprozeß des Knochens sind verbunden mit einer Kollagen-Neusynthese. Anschließend an die Synthese finden weitere intra- und extrazelluläre Kollagenprozesse statt. Unter Verwendung radioaktiver Kollagen-Vorstufen (¹⁴ C-Prolin) ist es möglich, die Kollagen-Syntheserate in einer Gewebekultur genau zu quantifizieren Somit ist es möglich, den Einfluß von Medikamenten auf die Kollagensynthese qualitativ und quantitativ zu erfassen.

Der Kollagen-Gesamtgehalt wird durch den sogenannten Hydroxyprolin-Test bestimmt. Die Aminosäure Hydroxyprolin kommt hauptsächlich im Kollagen vor, der Hydroxyprolin-Gehalt in Fremdproteinen ist zu vernachlässigen. Nach Freisetzung der Aminosäuren aus den Proteinen durch Hydrolyse (16 h bei 116° C, 22 % HCl) wird nach chemischer Modifizierung (Oxidation des 4-Hydroxyprolin zu Pyrrol) der Hydroxyprolin-Gesamtgehalt im Testansatz durch eine spezifische Farbreaktion mit p-Dimethylaminobenzaldehyd quantifiziert.

### 1.7 Bestimmung der Kollagen-Syntheserate

Kollagen in Gewebe und Medium wird nach der durch Proff (1991) modifizierten Methode von Miller und Roths (vgl. E.J. Miller und R.H. Roths "Methods in Symol." 82: 33 (1982)) gewonnen. Über mehrere Fällungsschritte mit anschließender Zentrifugation wird Kollagen über SDS-Gelelektrophorese und anschließendes Szintillationsmessen (Bestimmung der spezifischen Radioaktivität) quantifiziert. Zur Berechnung der Neusyntheserate wird der durch den Einbau von ¹⁴ C-Prolin bestimmte Kollagenanteil, bezogen auf den Kollagen-Gesamtgehalt, der durch den Hydroxyprolin-Test bestimmt wird, ermittelt.

Durch eine Quantifizierung der Kollagenbiosynthese ist es möglich, den Einfluß von Calciumhydroxid-Präparaten auf die Knochenbildung zu untersuchen.

Die Quantifizierung der Kollagenbiosynthese erfolgt - wie bei 1.7 angedeutet - nach der Technik der radioaktiven Markierung des Kollagens. Ein Bestandteil der Kollagenfaser ist die Aminosäure Prolin. Dem Kulturmedium wird eine genau definierte Menge von ¹⁴ C-markierten Prolins zugesetzt. Dieses Prolin wird in die während der Inkubation des Gewebes neu synthetisierten Proteine eingebaut. Nach der Trennung des Kollagens von anderen Proteinen ist es durch die Bestimmung der spezifischen Radioaktivitat möglich, eine exakte quantitative Aussage über die Neusyntheserate des Kollagens zu machen.

Die Isolierung des Kollagens geschieht über mehrere Fällungsschritte mit anschließender Zentrifugation und SDS-Gelelektrophorese. Bei der spezifischen Fällung des Kollagens werden die Kollagenfasern von anderen Proteinen getrennt, indem durch Zugabe von Natriumchlorid eine geeignete Salzkonzentration, bei der Nicht-Kollagen-Proteine zu einem großen Teil in Lösung bleiben, das Kollagen aber aus der Lösung als Niederschlag ausfällt, eingestellt wird. Durch anschließende Zentrifugation wird das Kollagen sedimentiert. Bei der SDS-Gelelektrophorese werden Proteine größenabhängig voneinander getrennt. Die Proteine wandern in einem elektrischen Feld durch eine Matrix aus einem hochvernetzten Polymer (Acrylamid). Kleine Proteine wandern schnell durch diese Matrix, da diese den Molekülen einen geringen Widerstand entgegensetzt, große Proteine wandern langsamer, da ihre Beweglichkeit durch die Matrix stark behindert wird. Nach Färbung werden Proteine in diesem Gel als sogenannte "Banden" sichtbar. Durch interne Größenstandards können auf diese Weise Proteine anhand ihrer Größe identifiziert werden.

Indem man interessierende Banden aus dem Gel herausschneidet, werden die Proteine einer weiteren Analyse, zum Beispiel einer Radioaktivitäts-Messung, zugänglich.

### Extraktion von Kollagen:

Durch Zugabe von 3 % Essigsäure wurde die Gewebekultur (vgl. 1.2) gestoppt. In Lösung gegangenes Kollagen wurde durch 2 M Natriumchlorid über Nacht bei 4° C gefällt und anschließend durch Zentrifugation (1 h, 24.000 g, 4° C) gewonnen. Das Sediment wurde in 10 ml 3 %iger Essigsäure aufgenommen. Durch mechanische Desintegration der Gewebeblöcke wird neusynthetisiertes Kollagen, das sich innerhalb des Gewebeblockes befindet, in die Analyse miteinbezogen. Gewebereste wurden durch Zentrifugation (1 h, 45.000 g, 4° C) abgetrennt. Das Kollagen im Überstand wurde durch eine weitere Fällung (2 M NaCl, 4° C) und Zentrifugation (1 h, 100.000 g, 4° C) gewonnen. Das Sediment wurde nach Solubilisierung gelelektrophoretisch aufgetrennt. Zur Kontrolle der Auftrennung der Proteine wurden sie im Gel angefärbt.

Das Gel wurde nach dem Lauf quer zur Laufrichtung in 5 mm breite Streifen zerschnitten, die Gelfragmente in Szintillationsgefäße überführt und im Szintillationszähler ausgezählt.

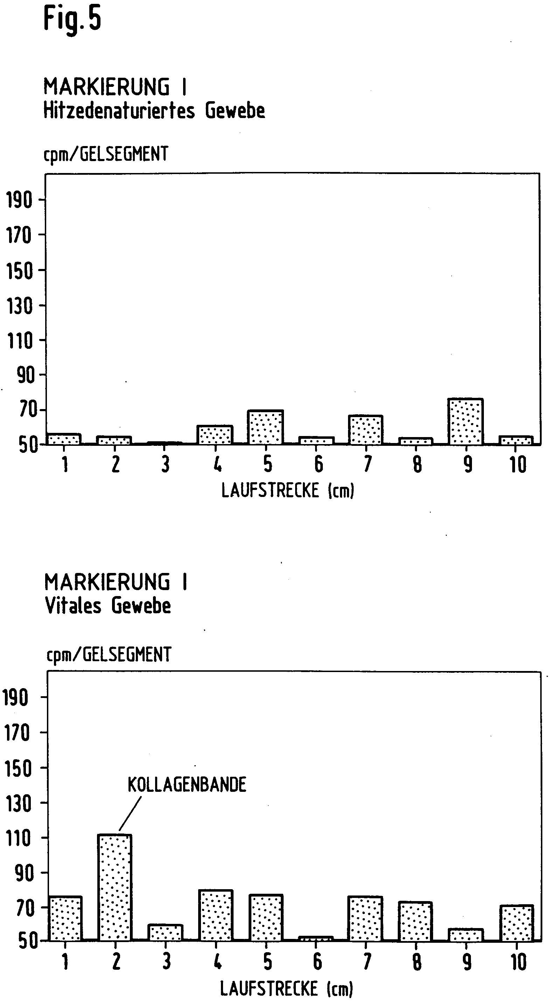

B) Die Figuren 5 bis 9 zeigen das Ergebnis einiger ausgewählter Experimente.
   Auf den Abbildungen ist die Radioaktivitäts-Verteilung im Gel gezeigt. Kollagen als relativ großes Protein ist in dem Bereich 2 cm vom Start entfernt zu finden.
   Der Kollagenbande, welche durch die Coomassie-Färbung nachweisbar wird, kann eine spezifische radioaktive Bande zugeordnet werden.
   In Fig. 5 ist der Vergleich zwischen einem vitalen und einem hitzedenaturierten Gewebe gezeigt.

- Fig. 5: Markierung I, Hüftkopf, Spongiosa, männlich, 45 Jahre;
Unterschied der Kollagensynthese-Leistung zwischen vitalem und hitzedenaturiertem Gewebe (CPM: Counts Per Minute). Gezeigt ist hier die Verteilung der Radioaktivität im Gel. Die Kollagenbande findet sich in einem Bereich, der etwa 2 cm vom Start entfernt liegt. Es tritt nur bei vitalem Gewebe eine Kollagenbande auf, das heißt der im Gel nachzuweisenden Radioaktivität entspricht eine Neusynthese des Kollagens während der Inkubation.

Das vitale Gewebe zeigt eine nachweisbare Kollagensynthese-Leistung, das abgetötete Gewebe zeigt dagegen keine Stoffwechselaktivität mehr. Dies zeigt, daß das nachgewiesene radioaktive Kollagen tatsächlich auf eine Kollagen-Neusynthese in der Gewebekultur und nicht auf eine unspezifische Bindung radioaktiven Prolins an Proteine des Knochengewebes zurückzuführen ist. Für alle Versuche wurde eine vergleichbare Menge Gewebematerial eingesetzt (ca. 100 mg).

Es sind weitere radioaktiv markierte Banden geringerer Größe nachweisbar, wobei es sich um Abbauprodukte des Kollagens handeln kann. Der Abbau von Kollagen in der Gewebekultur ist vor allem bei Markierungsexperimenten über 4 Tage Inkubationsdauer zu beobachten. Zu einem geringen Teil ist auch radioaktiv markiertes Prolin im Gel vorhanden, welches durch die Fällungen nicht vollständig entfernt werden konnte.

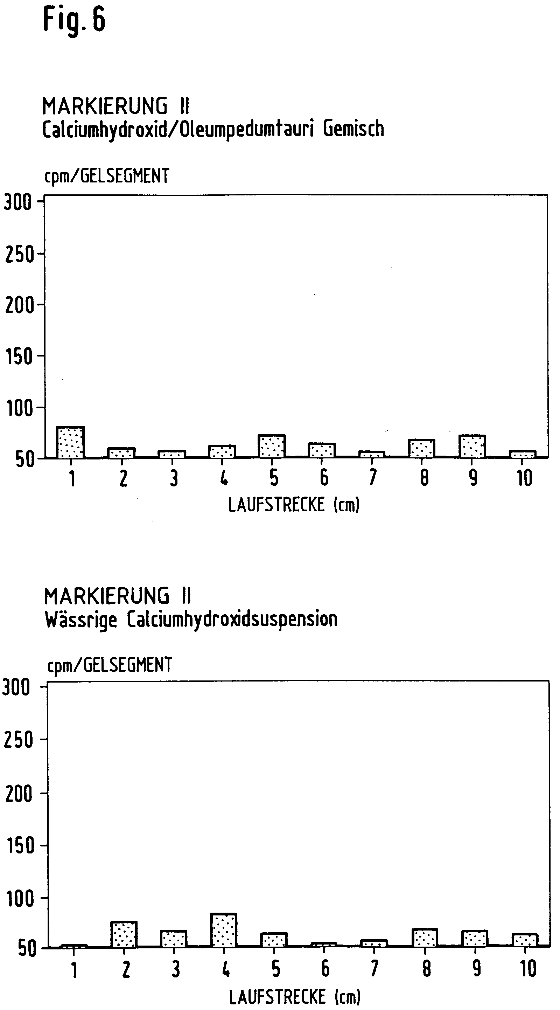

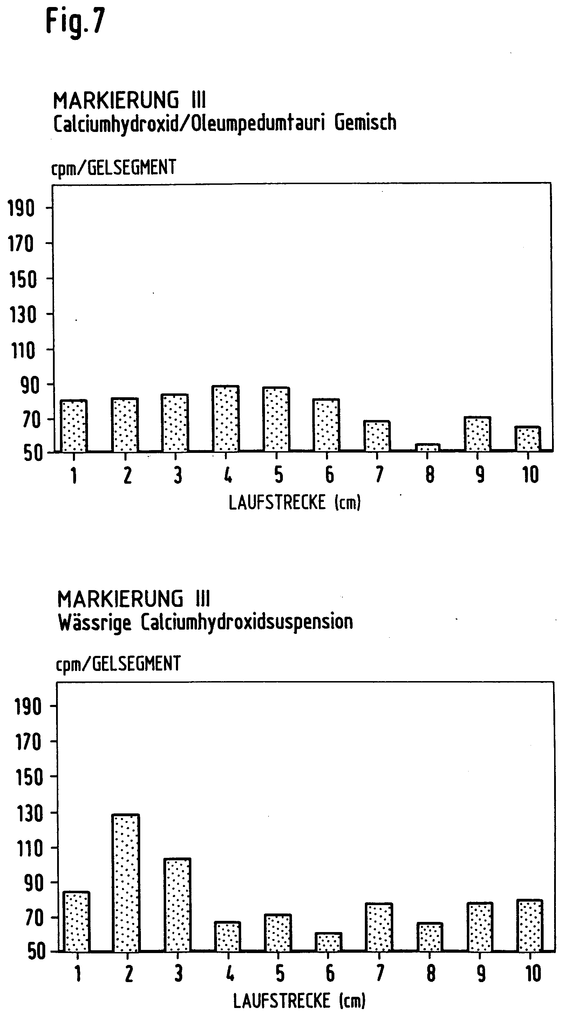

Die Figuren 6 bis 9 zeigen die Ergebnisse einiger ausgewählter Markierungsexperimente unter Calciumhydroxid-Einfluß. Es wurde die Kollagensynthese im unstimulierten Zustand (Kontrolle) unter Einfluß verschiedener Calciumhydroxid/Oleum pedum tauri-Gemische und eines wäßrigen Calciumhydroxid-Präparats bestimmt. In jeder Versuchsreihe wurde identisches Gewebe eingesetzt, das heißt Gewebeteile aus der gleichen Körperregion des Spenders. Im einzelnen zeigen die
- Fig. 6: Markierung II, Hüftkopf, Spongiosa, männlich, 75 Jahre;
Markierungsexperiment unter dem Einfluß eines Calciumhydroxid/Oelum pedum tauri-Gemischs und einer wäßrigen Calciumhydroxid-Suspension. Das Gewebe ist nur noch schwach stoffwechselaktiv. Gewebe von Patienten höheren Alters (über 65 Jahre) zeigt stets eine stark verminderte Kollagensynthese.
- Fig. 7: Markierung III, Hüftkopf, Spongiosa, männlich, 53 Jahre;
Markierungsexperiment unter dem Einfluß eines Calciumhydroxid/Oleum pedum tauri-Gemischs und einer wäßrigen Calciumhydroxid-Suspension. Die Inkubation mit dem Calciumhydroxid/Oelum pedum tauri-Gemisch zeigt keinen negativen Einfluß auf die Kollagensynthese, die Inkubation mit einer wäßrigen Calciumhydroxid-Suspension führte zu einer Nekrose des Gewebes.

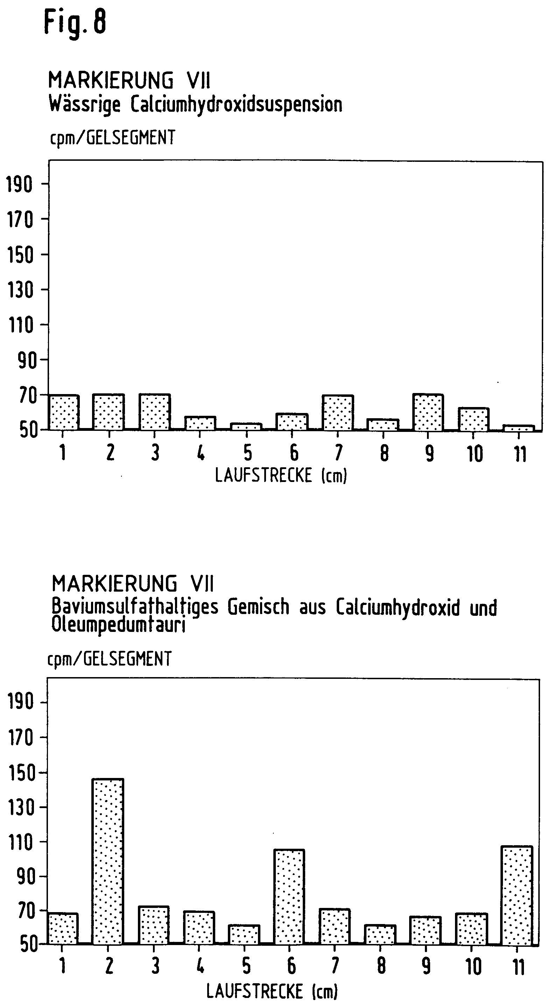

- Fig. 8: Markierung VII, Tibia-Kopf, Spongiosa, weiblich, 51 Jahre;
Markierungsexperiment unter dem Einfluß eines bariumsulfathaltigen Calciumhydroxid/Oleum pedum tauri-Gemischs und einer wäßrigen Calciumhydroxid-Suspension. Wie bei der Inkubation gemäß Fig. 7 ist auch hier nur bei Einsatz des Calciumhydroxid/Oleum pedum tauri-Gemischs eine Kollagensynthese-Leistung nachweisbar.

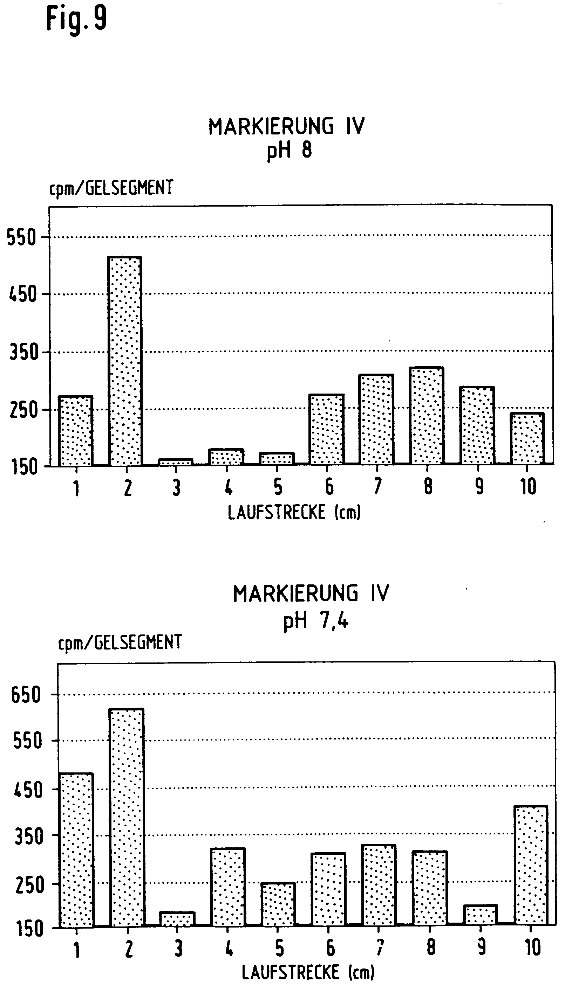

- Fig. 9: Markierung IV, Tibia-Kopf, Spongiosa, männlich, 63 Jahre;
Verteilung der Radioaktivität im Gel nach Inkubation bei pH 7, 4 und 8,0. Die Kollagensynthese ist nicht signifikant verändert, was die Toleranz des Knochengewebes gegenüber einer gemäßigten Alkalisierung zeigt.

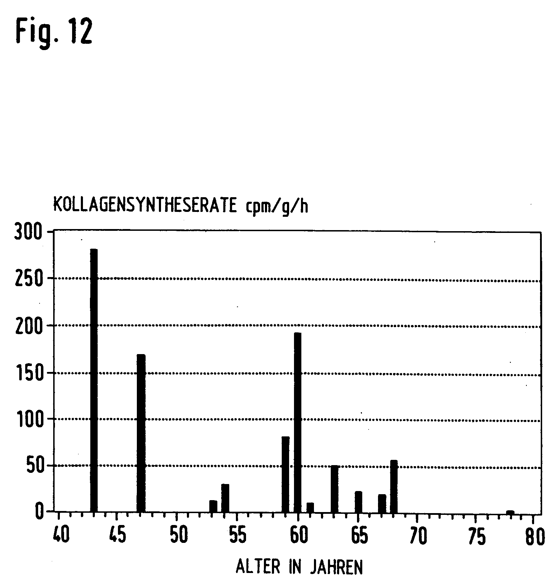

Aus den Markierungsexperimenten lassen sich folgende Aussagen treffen: Die Stoffwechselaktivität des Gewebes ist stark altersabhängig. Die Markierungsexperimente, bei denen Gewebe älterer Patienten eingesetzt wurde, zeigen eine geringere Kollagensynthese-Aktivität (Fig.6), die stimulierende Wirkung erfindungsgemäßer Gemische aus Calciumhydroxid und Oleum pedum tauri tritt jedoch nicht nur bei jungen, sondern auch bei älteren Patienten auf (Fig. 12).

Die Inkubation mit einer wäßrigen Calciumhydroxid-Suspension (Figuren 6 bis 8) führt in jedem Falle zu einer Nekrose des Gewebes. Eine Kollagensynthese ist nicht nachweisbar. Dies ist auf den starken pH-Anstieg in der Gewebekultur zurückzuführen. Nach dreitägiger Inkubation hat das Kulturmedium einen pH-Wert von 12 angenommen.

Unter dem Einfluß des erfindungsgemäßen Gemischs aus Calciumhydroxid und Oleum pedum tauri ist eine Kollagen-Neusynthese zu beobachten. Der pH des Mediums nach der Inkubation überschreitet nie einen Wert von 10,5. Signifikante Unterschiede zwischen verschiedenen Calciumhydroxid/Oleum pedum tauri-Gemischen (Figuren 7 und 8) traten nicht auf, wenn auch bei dem bariumsulfathaltigen Gemisch eine um etwa 10 % verminderte Kollagenneubildungsrate festzustellen ist. Auch in letzterem Falle ist die größte Menge radioaktiv markierter Proteine in der Kollagenbande zu finden.

Die Toleranz des Knochengewebes gegenüber schwach alkalischen pH- Werten ergibt sich aus einem Parallelexperiment zu dem in Fig. 5 erläuterten Experiment. Bei dem Parallelexperiment wurde der physiologische Hepes-Puffer des Kulturmediums (pH 7,4) durch einen Bicarbonat-Puffer (pH 8,0) ersetzt. Hierbei zeigte es sich, daß die Alkalisierung des Kulturmediums auf pH 8,0 keinen meßbaren Unterschied der Kollagensynthese-Leistung gegenüber pH 7,4 zur Folge hatte. Ab pH 8,5 ist keine gegenüber einer spontanen Kollagenneubildung erhöhte Kollagenneubildung mehr zu beobachten.

Die bislang gewonnenen Erkenntnisse, daß wäßrige Calciumhydroxid-Suspensionen aufgrund ihrer stark alkalisierenden Wirkung eine Nekrose des Gewebes herbeiführen und keine Kollagen-Neusynthese stimulieren, und daß andererseits Gemische aus Calciumhydroxid und Oleum pedum tauri eine signifikante stimulierende Wirkung auf die Kollagen-Neusynthese haben, wurden durch weitere Experimente erhärtet. Diese Versuche wurden - wie bereits beschrieben - durchgeführt.

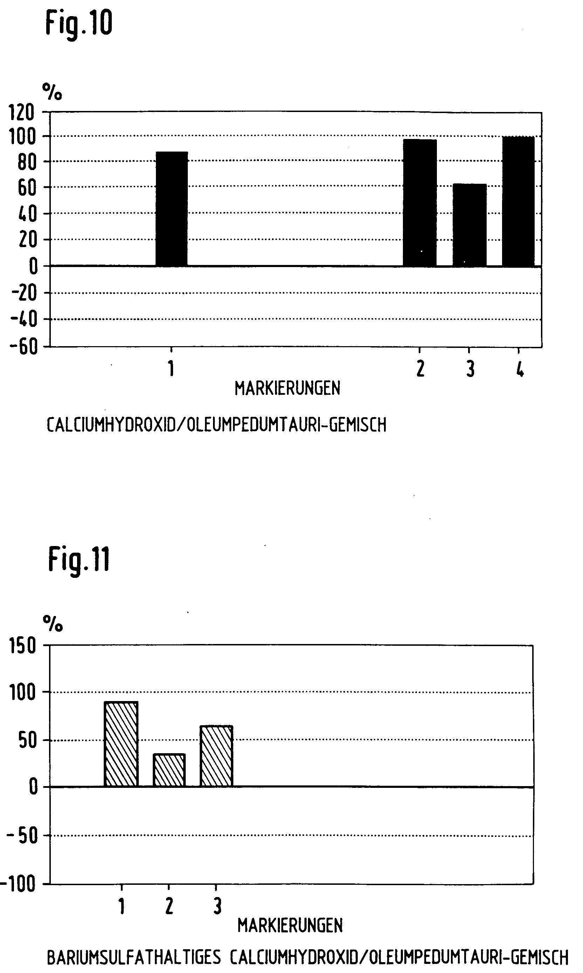

Die Abbildungen 10 und 11 zeigen die Menge neusynthetisierten Kollagens in den Versuchsansätzen mit einem Calciumhydroxid/Oleum pedum tauri-Gemisch im Vergleich zu Kontrollansätzen ohne dieses Gemisch. Die Differenz der neusynthetisierten Kollagenmenge in der Kontrolle und im Versuchsansatz ist in Prozent ausgedrückt. 0 % bedeutet keine gegenüber der Kontrolle vermehrte neusynthetisierte Kollagenmenge, Versuchsansatz mit dem Gemisch 100 % bedeutet eine gegenüber der Kontrolle um das zweifache erhöhte Kollagenneubildung unter Einfluß des Gemischs. Aus Fig. 10 geht hervor, daß bei den vier Experimenten unter dem Einfluß eines Calciumhydroxid/Oleum pedum tauri-Gemischs die Kollagensynthese gegenüber der Kontrolle zwischen 60 und 90 % erhöht ist. Diese Steigerung ist signifikant, da experimentell bedingte Schwankungen der Kollagensynthese-Leistung im Bereich von 10 bis 20 % liegen, die unter dem Einfluß des Calciumhydroxid/Oleum pedum tauri-Gemischs bestimmten Steigerungen dagegen zwischen 60 und 90 % liegen.

Die Abb. 11 zeigt die Ergebnisse bezüglich der Kollagensynthese-Leistung unter dem Einfluß eines bariumsulfathaltigen Gemischs aus Calciumhydroxid und Oleum pedum tauri. Wenn auch hier die Kollagensynthese-Leistung gegenüber dem bloßen Gemisch aus Calciumhydroxid und Oleum pedum tauri etwas verringert ist, kann die Anwesenheit von Bariumsulfat trotzdem erwünscht sein, und zwar dann, wenn das Calciumhydroxid-Präparat röntgensichtbar sein soll. Mit einem solchen Präparat können die mit der Behandlung einhergehenden Veränderungen und Resorptionen des Medikaments im Röntgenbild verfolgt werden.

Ergebnisse bei klinischer Anwendung des erfindungsgemäßen Gemischs nach Knochenfräsung bei Wurzelspitzenresektion bzw. bei der Präparation von Implantatbetten

### Beispiel 2

Wenn die Knochendefekte durch Fräsung mit dem erfindungsgemäßen Gemisch versorgt werden, gibt es Infektionsschutz, Entzündungshemmung (der normalen reaktiven Entzündung nach der Knochentraumatisierung) und eine starke osteoregenerierende Anregung. Bei 8 Zähnen mit Wurzelspitzenresektion und der apikalen Entstehung einer Knochenwunde von 0,5 bis 1 cm Durchmesser führte die Applikation des erfindungsgemäßen Gemischs zu einer starken Verminderung bzw. Freiheit von postoperativen Schmerzen. 3 Fälle waren völlig schmerzfrei: 4 Fälle zeigten nur am ersten Tag nach dem Eingriff leichte Beschwerden und ein Fall zeigte lediglich am 2. Tag nach dem Eingriff mittlere Schmerzen. Die normale zu erwartende starke Schwellung nach solchen Knochenfräsungen war in einem Falle überhaupt nicht vorhanden, in 6 Fällen war sie lediglich am 2. Tag leicht vorhanden. Nur ein Fall zeigte am 2. Tag starke Schwellung. In Bezug auf die postoperative Entzündung zeigten 2 Fälle überhaupt keine Entzündungserscheinungen; 3 Fälle wiesen eine leichte Entzündung am 1. bzw. 2. oder 3. Tag auf. 3 Fälle zeigten nach dem 1. bzw. 2. Tag eine leichte bis mittlere Entzündung auf. In keinem Falle gab es die üblich zu beobachtende starke postoperative Entzündung.

### Beispiel 3

Bei einer 50-jährigen zahnlosen Patientin im Oberkiefer wurden 7 Knochenbohrungen zur Aufnahme von Implantaten gefräst. Die Knochenbohrungen waren 1,5 bis 2 cm tief und etwa 3 mm breit. Die Knochenbohrungen wurden vor Einschrauben der Implantate mit dem erfindungsgemäßen Gemisch gefüllt. Das erstaunliche Ergebnis war, daß die Patientin am nächsten Tag nach dem Eingriff keine Schmerztabletten brauchte, die Schwellung war minimal. Besonders auffallend ist die Tatsache, daß nach 2 Wochen im Röntgenbild die zu erwartende reaktive Osteolyse rings um die Implantate völlig fehlte. Dies bedeutet, daß schon nach 2 Wochen eine Osteointegration der Implantate zu beobachten ist, die normalerweise erst in 5-6 Monaten zu erwarten ist. Das nach 2 Wochen nach der Knochenfräsung zu beobachtende Röntgenbild entspricht zu 90 % dem zu erwartenden Röntgenergebnis nach 6 Monaten.

## Patentansprüche

1. Verwendung eines Gemischs aus Calciumhydroxid und Oleum pedum tauri bei der Herstellung eines Medikaments zur in vivo-Stimulierung der Kollagenneubildung.

2. Verwendung eines Gemischs nach Anspruch 1, in dem das Volumenverhältnis Calciumhydroxid/Oleum pedum tauri 2/1 bis 1/2 beträgt.

3. Verwendung eines Gemischs nach Anspruch 1, in dem das Volumenverhältnis Calciumhydroxid/Oleum pedum tauri 1/1 beträgt.

4. Verwendung eines Gemischs nach einem der Ansprüche 1 bis 3, das Bariumsulfat enthält.

5. Verwendung eines Gemischs nach einem der Ansprüche 1 bis 4, das weiße Vaseline enthält.

## Claims

1. Use of a mixture of calcium hydroxide and oleum pedum tauri in a method for the production of a medicament for stimulating collagen reformation in vivo.

2. Use of a mixture of Claim 1 wherein the volume ratio of calcium hydroxide oleum pedum tauri is from 2/1 to 1/2.

3. Use of a mixture of Claim 1 wherein the volume ratio of calcium hydroxide oleum pedum tauri is 1/1.

4. Use of a mixture of Claim 1 to 3 containing barium sulfate.

5. Use of a mixture of Claims 1 to 4 containing white petroleum jelly.

## Revendications

1. Utilisation d'un mélange constitué d'hydroxyde de calcium et d'oleum pedum tauri, dans la fabrication d'un médicament pour stimulation in vivo du renouvellement des collagènes.

2. Utilisation d'un mélange selon la revendication 1, dans lequel le rapport en volume hydroxyde de calcium/oleum pedum tauri est compris dans la plage allant de 2/1 à 1/2.

3. Utilisation d'un mélange selon la revendication 1, dans lequel le rapport en volume hydroxyde de calcium/oleum pedum tauri est de 1/1.

4. Utilisation d'un mélange selon l'une des revendications 1 à 3, contenant du sulfate de baryum.

5. Utilisation d'un mélange selon l'une des revendications 1 à 4, contenant de la vaseline blanche.
